# EUROPÄISCHE PATENTANMELDUNG

(11) **EP 2 100 634 A2**
(43) Veröffentlichungstag der Anmeldung: **16.09.2009**
(21) Anmeldenummer: 09159760.9
(22) Anmeldetag: 18.07.2007
(51) Int. Cl.: A61M 15/00

(54) **Blisterpackung**

(30) Priorität: 26.09.2006 DE 102006045788
(62) Teilanmeldung aus: 07787672.0
(71) Anmelder: von Schuckmann, Alfred, 47627 Kevelaer (DE)
(72) Erfinder: von Schuckmann, Alfred, 47627 Kevelaer (DE)
(74) Vertreter: Rieder, Hans-Joachim

(57) **Zusammenfassung**

Die Erfindung betrifft eine zur Verwendung in Medikamenten-Spendern geeignete Blisterpackung (25) mit auf einer Aluminiumschicht (28) kreisförmig auflaminierten Kavitäten (26) und schlägt zur Verbesserung der Handhabbarkeit vor, dass die die Kavitäten (26) tragende Alu-Schicht (28) die Gestalt eines Ringstreifens hat.

## Beschreibung

Die Erfindung betrifft eine Medikamenten-Blisterpackung gemäß Gattungsbegriff des Hauptanspruches.

Blisterpackungen der in Rede stehenden Art sind z.B. durch die US-PS 4.778.054 bekannt. Die dortige Lösung trägt in einem Gehäuse eine scheibenförmige, karussellartig schrittweise weiter schaltbare Blisterpackung. Diese besitzt eine Mehrzahl von in kreisförmiger Anordnung vorgesehene kuppelförmige Kavitäten. In jeder Kavität ist eine Portion der pulverförmigen Masse aufgenommen. Verschlossen sind die gefüllten Kavitäten durch eine auflaminierte Aluminiumfolie. Zur Inhalation wird die jeweils in die Entleerungsposition gebrachte Kavität mittels einer Nadel geöffnet, welche Nadel die den Kavitätenboden formende Aluminiumfolie und die Kuppel-Decke durchstößt. Nach Rückzug der Nadel liegt die nunmehr geöffnete Kavität in einem zum Mundstück führenden Kanal, zur Entleerung über einen Saugstrom. Die möglichst gleichmäßige und vollständige Entleerung ist problematisch, insbesondere sogar vom Füllungsgrad der Kavität abhängig. Die Handhabung ist ungünstig.

Im Hinblick auf den zuvor beschriebenen Stand der Technik besteht die technische Problematik der Erfindung darin, eine Blisterpackung vorzuschlagen, die in einem Spender der in Rede stehenden Art insbesondere hinsichtlich der Entleerung der Kavitäten verbesserte Bedingungen bringt.

Diese Problematik ist zunächst und im Wesentlichen durch den Gegenstand des Anspruches 1 gelöst, wobei darauf abgestellt ist, dass die Blisterpackung als Ring gestaltet ist, so dass die Durchstoß-Nadel in Parallelbewegung zur Aluminiumschicht durch die Kuppelwände fahren kann. Die Aluminium-Schicht bleibt unverletzt. Der Durchstoß kann sehr nahe der Alu-Schicht erfolgen. Stößt die Nadel vom Inneren des Ringstreifen durch, so ist nicht nur eine sehr raumsparende Bauform erreichbar und das Durchstoßen erfolgt in einem auch bei dünner Kavitätenwand genügend stabilen Zone, die vollständige Entleerung erlaubt. Die beiden gestochenen, nach Rückhub der Nadel offenen Löcher liegen im Wege eines Saugluftstrom-Kanals zum Mundstück. Entsprechend wird zur Entleerung einer Kavität die Kuppel stets mittels der Nadel zweifach durchstochen. Die den Kavitätenboden formende Aluminiumfolie bleibt hiervon unberührt; sie bleibt unbeschädigt. Darüber hinaus reißen so geformte Löcher bedingt durch die Materialwahl nicht oder nicht wesentlich über den Nadeldurchmesser hinaus auf, was eine entsprechend maßlich geringe Auslappung des Öffnungsrandes zur Folge hat. Einer Art Taschenbildung, in welcher sich im Zuge der Entleerung pulverförmige Masse verstecken kann, wird wirksam verhindert. Ein weiterer Vorteil ergibt sich durch die Anordnung beider Löcher im Bereich der Kuppel. Hieraus ergibt sich im Zuge der Inhalation durch Einatmen ein bspw. sekantenartig die Kuppel durchsetzender Saugluftstrom, der quer durchströmend und sich unter der Kuppel wirbelnd verteilend zu einem völligen Entleeren der Kavität führt.

Nach Leerung einer Kavität wird der Blisterpackungs-Ring, bevorzugt unter Zuhilfenahme eines Trägers um einen Schritt weiter geschaltet, wonach die nächste Kavität in die Wirkstellung gelangt. Hierzu ist ein Schrittschaltwerk vorgesehen, das die kuppelförmigen Kavitäten schrittweise in die Entleerungsposition bewegt.

Nachstehend ist die Erfindung anhand der beigefügten Zeichnung, welche lediglich ein Ausführungsbeispiel darstellt, näher erläutert. Es zeigt:
- Fig. 1: in perspektivischer Vorderansicht den Spender bei verschlossenem Mundstück;

- Fig. 2: eine weitere perspektivische Darstellung des Spenders gemäß Fig. 1
- Fig. 3: die perspektivische Rückansicht hierzu;
- Fig. 4: eine der Fig. 1 entsprechende perspektivische Darstellung, jedoch nach Verschwenken der Verschlusskappe zur Freigabe des Mundstückes;

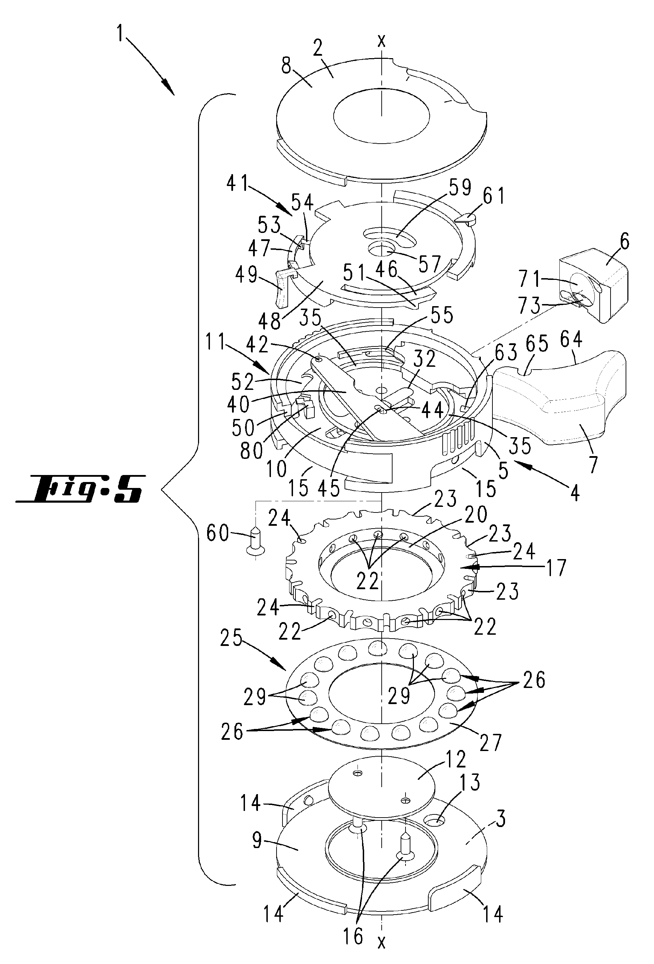

- Fig. 5: eine perspektivische Explosionsdarstellung des Spenders;

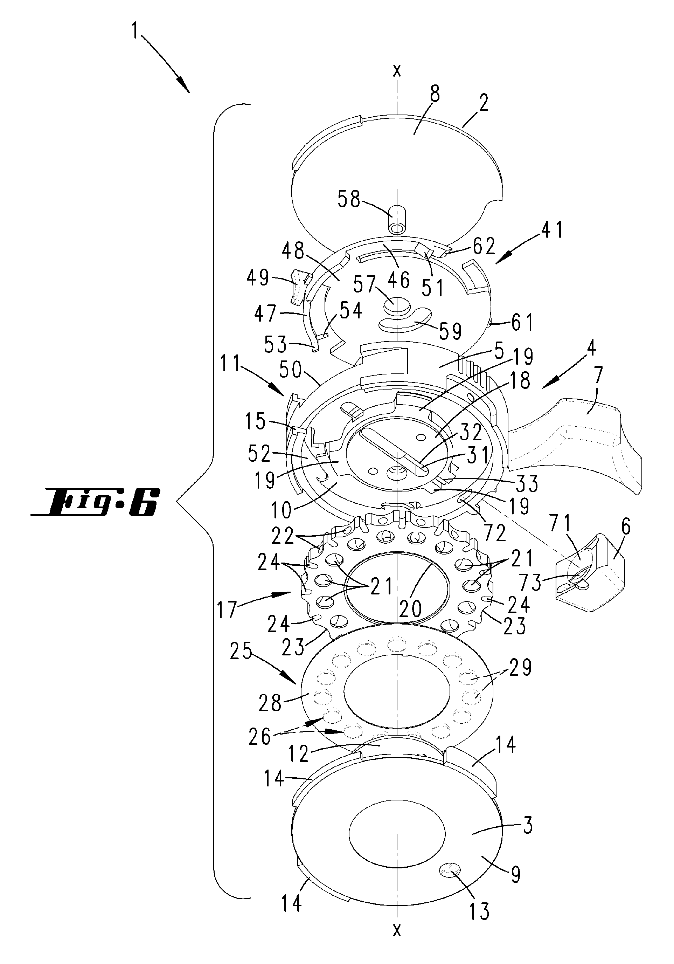

- Fig. 6: eine weitere Explosionsdarstellung mit Blickrichtung auf Unterseiten der Spenderteile;

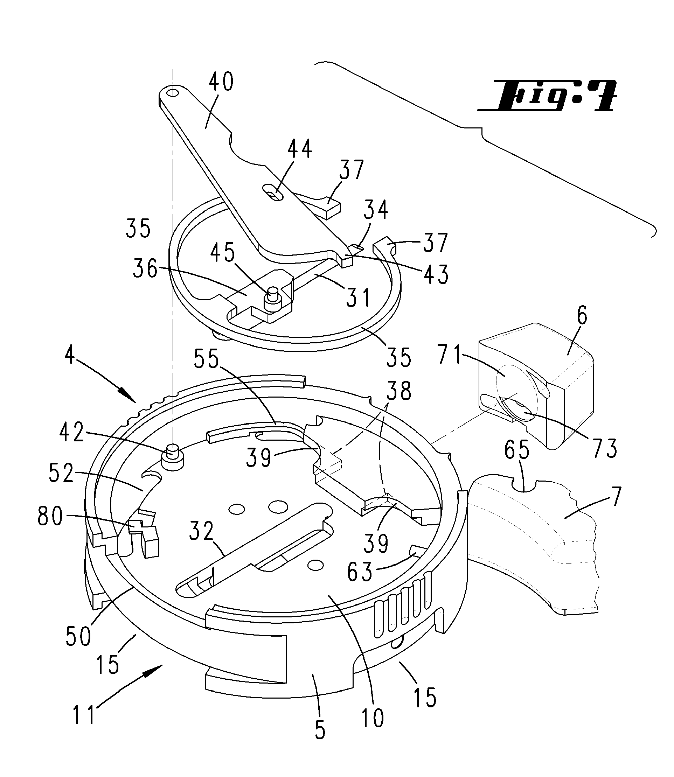

- Fig. 7: eine Teil-Explosionsdarstellung des Spendergrundgehäuses, des Mundstückes, einer mit einer Feder versehenen Nadel sowie einem Schleppteil;

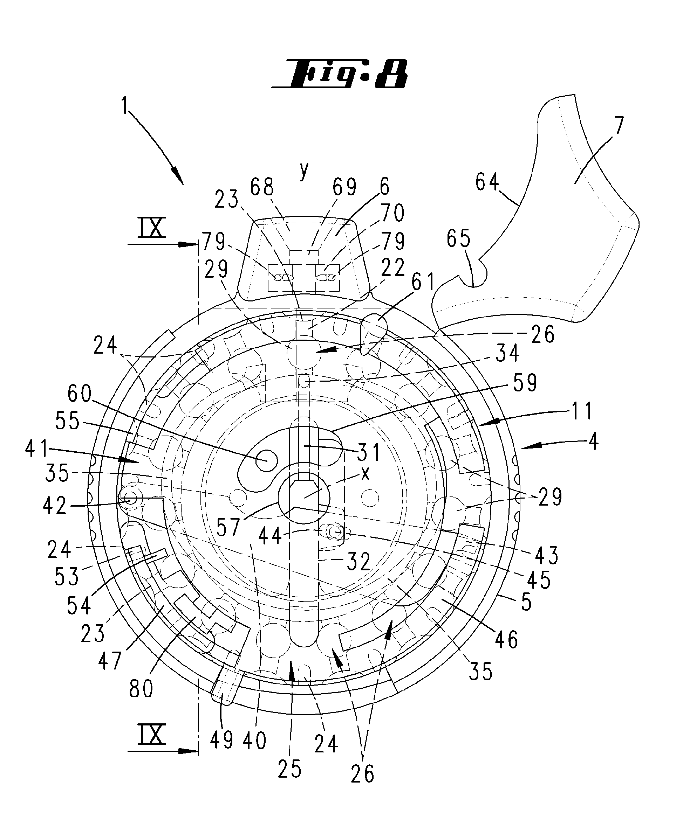

- Fig. 8: eine Vorderansicht gegen den Spender in Bereitschaftsstellung unter Fortlassung eines Deckels;

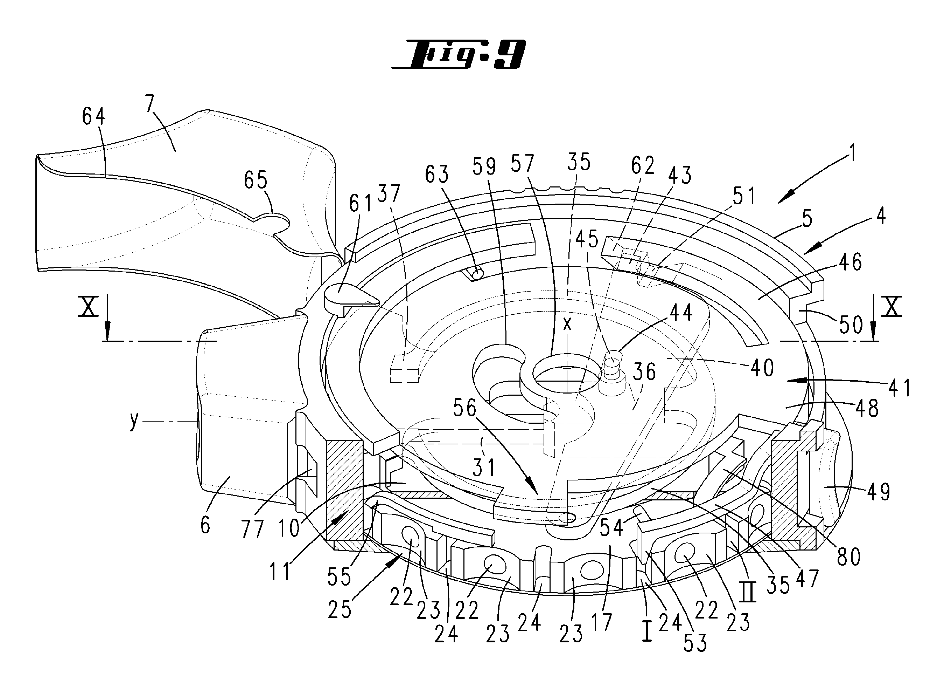

- Fig. 9: den Spender in partiell geschnittener, perspektivischer Darstellung, gleichfalls unter Fortlassung des Deckels, die Bereitschaftsstellung betreffend;
- Fig. 10: den Längsschnitt gemäß der Linie X-X in Fig. 9;
- Fig. 11: eine der Fig. 9 entsprechende Darstellung, jedoch im Zuge einer handbetätigten Nadelverlagerung zur Freilegung einer Kavität,
- Fig. 12: eine Folgedarstellung zur Fig. 11;

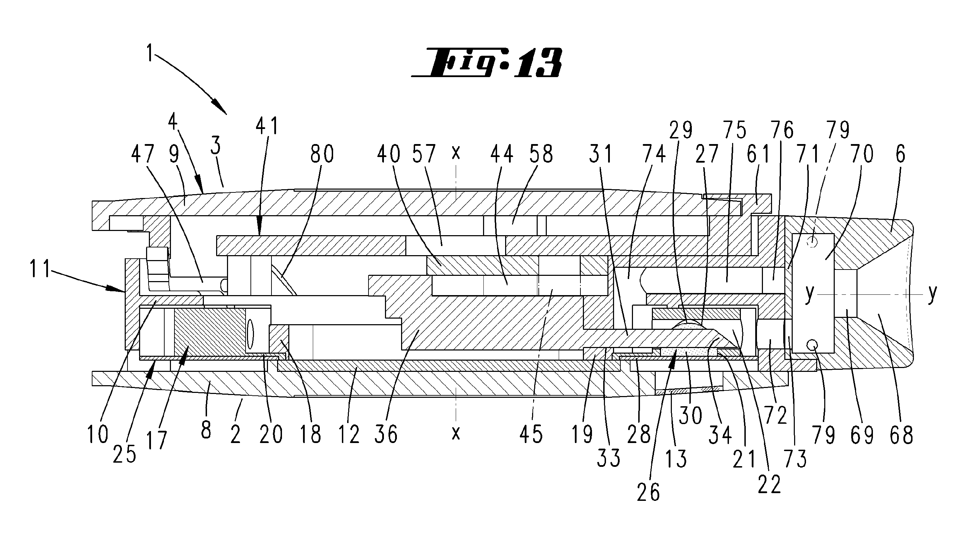

- Fig. 13: eine Schnittdarstellung gemäß Fig. 10, jedoch die Stellung bei Durchstoßen der kuppelförmigen Kavität betreffend;

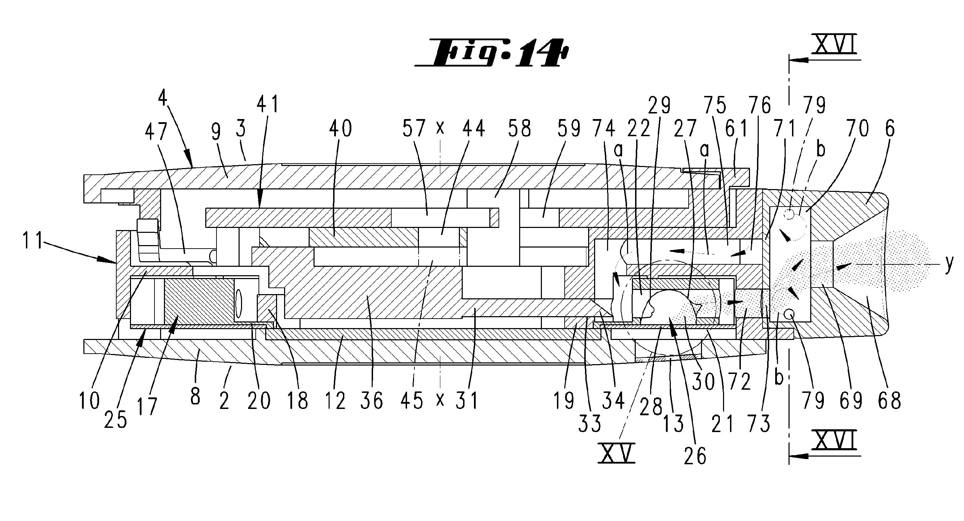

- Fig. 14: eine Folgedarstellung zu Fig. 13 nach Durchstoßen und Rückverlagerung der Nadel in die Grundstellung sowie bei angedeutetem Inhalationsvorgang;
- Fig. 15: die Herausvergrößerung gemäß dem Bereich XV in Fig. 14;

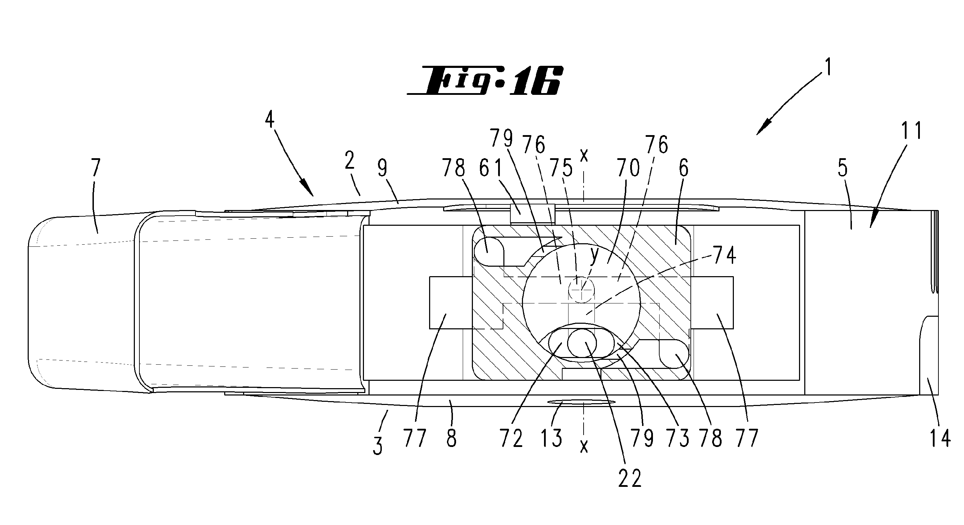

- Fig. 16: den Schnitt gemäß der Linie XVI-XVI in Fig. 14;
- Fig. 17: eine weitere perspektivische Darstellung gemäß Fig. 9, jedoch eine Folgedarstellung zu Fig. 12 betreffend bei nach einer Inhalation erfolgter manueller Schrittschaltwerkbetätigung;

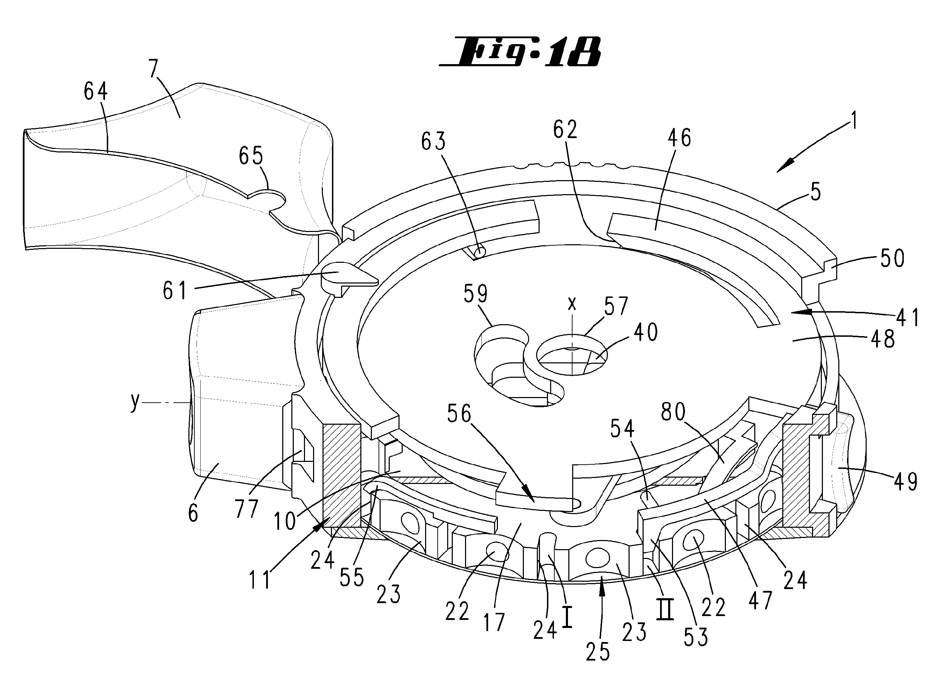

- Fig. 18: eine Folgedarstellung zu Fig. 17, die erreichte Grundstellung betreffend;
- Fig. 19: in perspektivischer Darstellung das Mundstück und der dem Mundstück zuzuordnende Gehäusebereich zur Darstellung der Strömungswege;
- Fig. 20: in perspektivischer Einzeldarstellung die Nadel mit angeformten Federn.

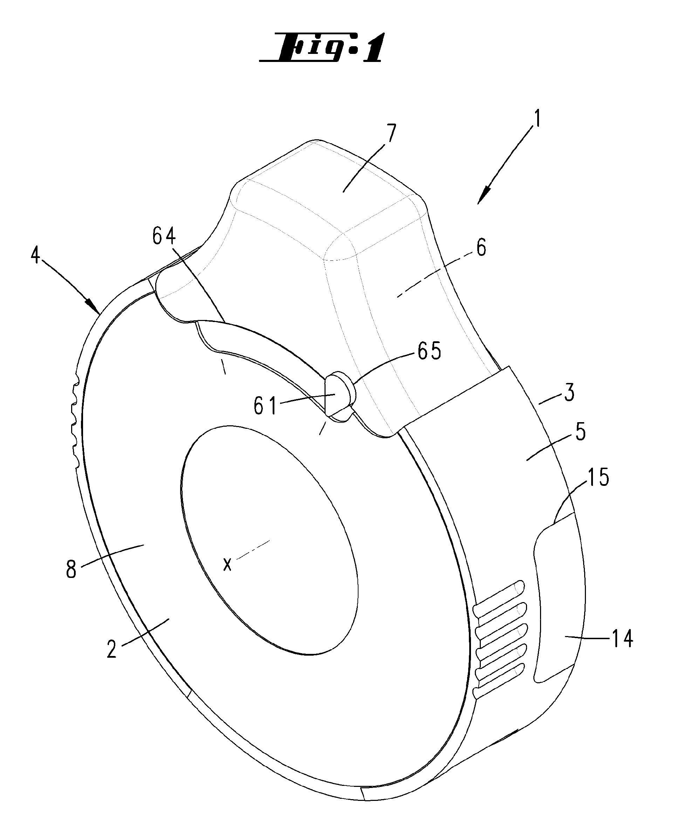

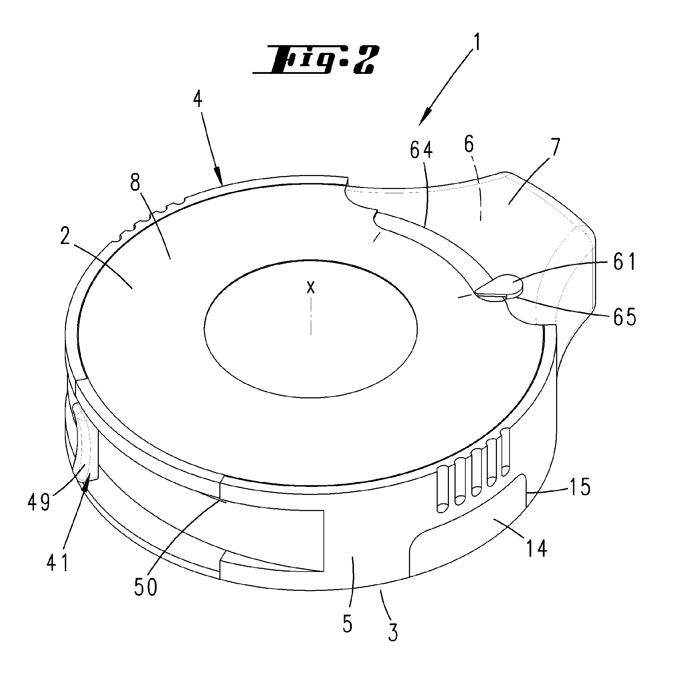

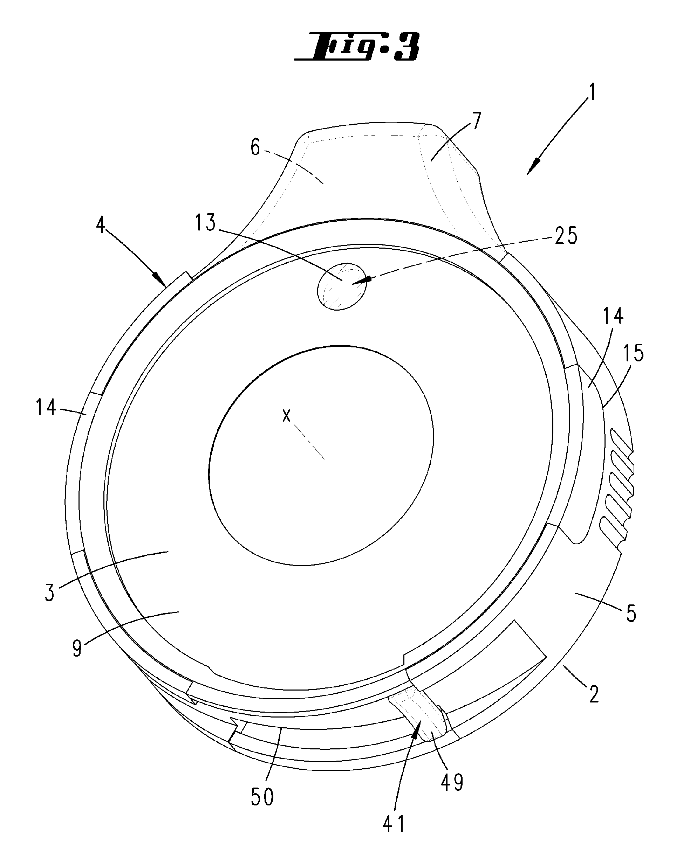

Dargestellt und beschrieben ist zunächst mit Bezug zu den Fig. 1 bis 4 ein Spender 1 für pulverförmige Massen, insbesondere zur Inhalation geeigneter Medikamenten. Der Spender 1 weist bei insgesamt handlichem Format einen im Wesentlichen kreisrunden Grundriss auf mit einer kreisscheibenförmigen Vorderseite 2 und einer im Grundriss gleichgestalteten Rückseite 3. Diese sind unter Aufnahme einer gemeinsamen Spenderachse x in Achsrichtung zueinander beabstandet. Die das Spendergehäuse 4 radial außen begrenzende Mantelwand 5 erstreckt sich entlang der zugewandten Randkanten der Vorder- und Rückseite, dies mit einem in Axialrichtung betrachteten Stärkenmaß, welches etwa einem Drittel bis einem Viertel des Spenderdurchmessers entspricht.

Aus der insgesamt etwa kreisscheibenförmigen Gestaltung des Gehäuses 4 wächst etwa über ein Achtel der Umfangslänge betrachtet radial nach außen ein Mundstück 6 aus. Über dieses erfolgt die Inhalation durch Einatmen.

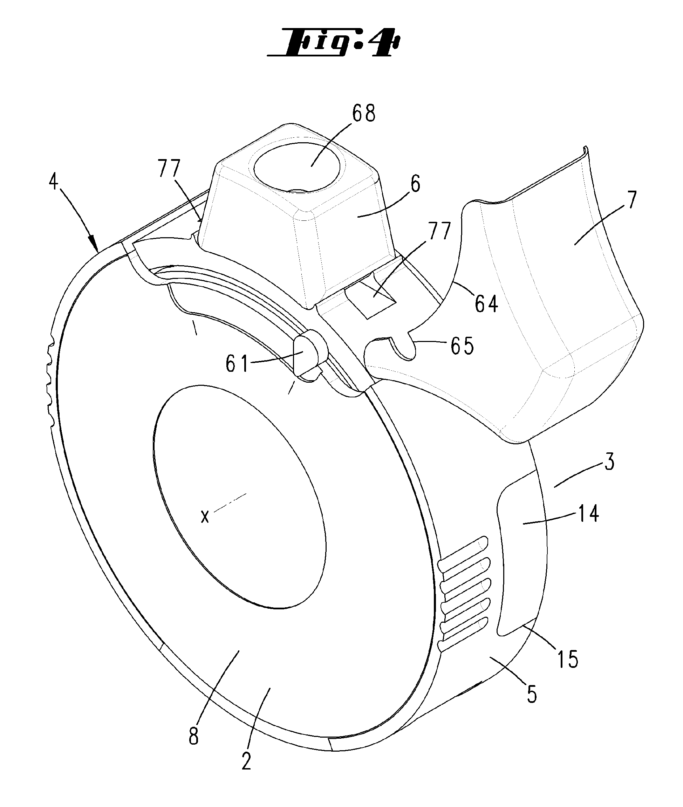

Das Mundstück 6 ist durch eine abschwenkbare Verschlusskappe 7 verschließbar, welche Verschlusskappe 7 an der Mantelwand 5 des Gehäuses 4 anscharniert ist. Die Scharnierachse verläuft parallel versetzt zu der Gehäuseachse x, so dass die Verschwenkung der Verschlusskappe 7 orientiert an die Umfangsrichtung des Gehäuses 4 erfolgt. Die Anscharnierung ist in Form eines Firmscharniers gewählt. Dieses ist so ausgelegt, dass die Verschlusskappe 7 in der Offenstellung gemäß der Darstellung in Fig. 4 in eine Selbsthaltung tritt. In der Verschlussstellung, bspw. in Fig. 1 dargestellt, ist die Verschlusskappe 7 in einer Raststellung gehalten, so dass ein Öffnen derselben nur willensbetont möglich ist.

Das Gehäuse 4 setzt sich im Wesentlichen zusammen aus einer die Vorderseite 2 bildenden, scheibenförmigen Deckwandung 8, einer mit demselben Durchmesser versehenen Deckwandung 9 zur Bildung der Rückseite 3 und der erwähnten im Grundriss kreisringförmigen Mantelwand 5. Letztere ist in Dickenrichtung - in Ausrichtung der Achse x betrachtet - mittig mit einem Zwischenboden 10 versehen. Dieser ist einstückig mit der Mantelwand 5 gebildet.

Die vorderseitige Deckwandung 8 und das Gehäuse-Mittelteil 11 mit Zwischenboden 10 und Mantelwand 5 sind nach Montage bevorzugt nicht mehr betrieblich voneinander trennbar. Die die Rückseite 3 definierende Deckwand 9 hingegen ist zur Freilegung des überdeckten Raumes entfernbar an dem Gehäuse-Mittelteil 11 gehaltert, so insbesondere randseitig rastgeschaltet.

Die Deckwandung 9 ist weiter in einem radial äußeren Bereich mit einem transparenten Bereich zur Bildung eines Sichtfensters 13 versehen. Letzteres ist bei festgelegter Deckwandung 9 an dem Gehäuse-Mittelteil 11 zugeordnet dem Mundstück 6 orientiert positioniert. Die Deckwandung 9 verfügt zur positionsrichtigen Anordnung an dem Gehäuse-Mittelteil 11 über leistenförmige Eingriffsmittel 14, die in entsprechende randoffene Ausnehmungen 15 im Bereich der Mantelwand 5 eingreifen und keine andere Positionierung erlauben. In diesen Eingriffbereichen ist die Rastfestlegung verwirklicht.

Das Sichtfenster 13 kann bei insgesamter Ausführung des Spenders 1 aus Kunststoffspritzteilen bspw. im Zuge eines Zwei-Komponenten-Spritzverfahrens ausgebildet sein, bei welchem in dem das Sichtfenster 13 darstellenden Bereich ein transparenter Kunststoff verwendet wird. Das Sichtfenster 13 kann darüber hinaus je nach Auslegung der transparenten Fläche einen lupenartigen Effekt erzeugen.

Der Zwischenboden 10 trennt das Gehäuse-Mittelteil 11 im Wesentlichen in einen von der betrieblich nicht lösbaren Deckwandung 8 überfangenen und somit geschützt liegenden Mechanikbereich und einen der lösbaren Deckwand 9 zugeordneten Massenträger-Bereich ein. In Letzterem ist ein kreisringförmiger Träger 17 aufgenommen. Dieser weist eine in Axialrichtung gemessene Stärke auf, die etwas geringer bemessen ist als die lichte Höhe zwischen der zugewandten Oberfläche des Zwischenbodens 10 und der diesen Bereich überfangen Deckwandung 9.

Der Träger 17 ist radial innen in Umlaufrichtung um die Achse x geführt. Hierzu weist der Zwischenboden 10 zunächst eine zentrale kreiszylinderartige Erhöhung 18 auf, von welcher über den Umfang betrachtet drei gleichmäßig zueinander beabstandete Radialvorsprünge 19 ausgehen, die insgesamt einen gegenüber der Erhöhung 18 vergrößerten Kreisdurchmesser definieren. Letzterer spiegelt sich im Wesentlichen wider in dem Innendurchmesser des Trägers 17, demzufolge der Träger 17 entlang der Radialaußenflächen der Erweiterungen 19 geführt ist.

Abgewandt dem Zwischenboden 10 ist der Träger 17 entlang der radial inneren Randkante mit einem nach radial innen überragenden Ringkragen 20 versehen, mit welchem der Träger 17 zugleich auf der zentralen Erhöhung 18 entlang der umlaufenden Randkante aufliegt. Dieser Radialkragen 20 ist in Dickenrichtung, d. h. betrachtet in Ausrichtung der Achse x, wesentlich materialverjüngt, weist entsprechend eine Stärke auf, die etwa einem fünfzehntel bis einem zwanzigstel der entsprechenden Trägerstärke entspricht.

Oberflächenseitig, im Einbauzustand zugewandt der den Träger 17 überdeckenden Deckwandung 9 ist der Träger 17 mit Aufnahmebohrungen 21 versehen. In dem dargestellten Ausführungsbeispiel sind dies insgesamt 15 umlaufend gleichmäßig zueinander beabstandete, gleiche Durchmesser aufweisende und auf einer gemeinsamen Durchmesserlinie angeordnete Aufnahmebohrungen 21. Jede dieser Aufnahmebohrungen 21 mündet in einem Querkanal 22. Die gleichfalls insgesamt fünfzehn Querkanäle 22 sind voneinander getrennt und jeweils streng radial zur Achse x ausgerichtet, wobei weiter jeder Querkanal 22 sowohl nach radial innen als auch nach radial außen frei mündet.

Jeder radial äußere Mündungsbereich der Querkanäle 22 ist in einem Grundriss betrachtet konkav gekrümmt, so dass die Mündungsöffnung des Querkanals 22 mit Bezug auf die äußere Durchmesserlinie des Trägers 17 radial zurückversetzt ist. Der Durchmesser der Konkavbereiche 23 ist so gewählt, dass zwischen zwei benachbarten Querkanälen 22 jeweils ein Abschnitt ursprünglichen Trägerdurchmessers verbleibt.

In diesen zwischen zwei Querkanälen 22 verbleibenden, den ursprünglichen Trägerdurchmesser aufweisenden Bereichen sind jeweils radial nach innen weisende Ausklinkungen 24 ausgeformt. Diese sind in Form von Radialschlitzen gebildet. Der Außendurchmesser des Trägers 17 entspricht im Wesentlichen dem Innendurchmesser der Mantelwand 5 des Gehäuse-Mittelteils 11.

Der Träger 17 dient zur Aufnahme und Transport einer Blisterpackung 25. Diese ist ähnlich dem Träger 17 kreisringförmig gebildet mit einem an dem Innendurchmesser der Mantelwand 5 angepassten Außendurchmesser. Der Innendurchmesser der Blisterpackung 25 entspricht im Wesentlichen dem des Trägers 17.

In üblicher Weise besteht die Blisterpackung 25 aus einem, Kavitäten 26 bildenden Kunststoff-Ringteil 27 und einer auflaminierten Schicht 28 aus einer Aluminium Folie. Die Kavitäten 26 werden jeweils geformt durch im Wesentlichen halbkugelförmige, aus dem Kunststoffmaterial herausgeformte Kuppeln 29. In jeder Kavität 26 ist die zu inhalierende Masse 30 vorportioniert aufgenommen. Die auflaminierte Aluminiumfolienschicht 28 bildet den Kavitätenboden aus. Entsprechend der Anzahl von Aufnahmebohrungen 21 ist die Blisterpackung 25 mit Kavitäten 26 versehen, so in dem dargestellten Ausführungsbeispiel mit fünfzehn derartiger Kavitäten 26, die auf einer gemeinsamen Durchmesserlinie umfangsmäßig gleichmäßig zueinander beabstandet angeordnet sind. Der jeweilige Kuppeldurchmesser entspricht im Wesentlichen dem Durchmesser einer Aufnahmebohrung 21.

Die Blisterpackung 25 wird mit den Kavitäten 26 dem Träger 17 zugewandt durch Einlegen der Kuppeln 29 in die Aufnahmebohrungen 21 des Trägers 17 formschlüssig und somit drehfest zum Träger 17 festgelegt. Jede Kavität 26 bzw. jede Kuppel 29 ragt hierbei die Aufnahmebohrung 21 durchsetzend in den jeweiligen Querkanal 22 des Trägers 17 ein, diesen Querkanal 22 bei unversehrter, gefüllter Kuppel 29 im Wesentlichen verschließend.

Zugeordnet jeder Kavität 26 ist die Blisterpackung 25 auf der freien Oberfläche der Aluminiumfolienschicht 28 mit einer nicht dargestellten Kennung versehen, so beispielsweise anfangend bei fünfzehn Kavitäten 26 mit der Ziffer 15 und über die weiteren Kavitäten 26 abwärts zählend, welche Kennung durch das Sichtfenster 13 visuell erfassbar ist. So ist dem Benutzer insbesondere eine Information über die Anzahl noch vorhandener Inhalationsportionen gegeben.

Das Blister 25 wird mittels eines scheibenförmigen Festlegungsteils 12 unter Einklemmung des verdünnten radial inneren Ringkragens 20 des Trägers 17 an der zentralen Erhöhung 18 des Zwischenbodens 10 gehaltert, wozu Formschlussverbindungsmittel 13 das Teil 12 durchsetzend in die Erhöhung 18 eingreifen. Letztere können auch einstückig an dem Festlegungsteil 12 angeformt sein.

Nach Abheben der rastgehalterten Deckwandung 9 und Entfernen des Festlegungsteils 12 in das Blister 25 zum Austausch freigelegt.

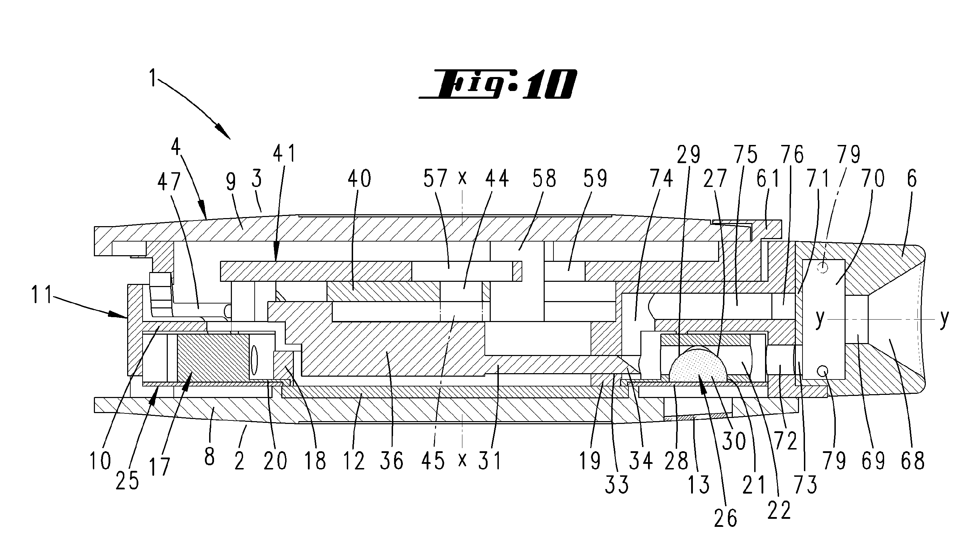

Zur Ausgabe der in jeder Kavität 26 bevorrateten Masse 30 ist zunächst ein Öffnen der Kavität 26 Voraussetzung. Dieses Öffnen erfolgt bei dem vorgeschlagenen Spender 1 durch Lochung, so erfindungsgemäß durch Lochen der in Bereitschaftsstellung befindlichen Kuppel 29. Hierzu ist eine in die Lochungsstellung bringbare Nadel 31 vorgesehen. Diese liegt in einer radial ausgerichteten, schlitzartigen Ausnehmung 32 des Zwischenbodens 10 ein, welche schlitzartige Ausnehmung 32 in Erstreckungsrichtung betrachtet die Achse x querend sich unter Belassung eines geschlossenen Randabschnittes über einen Großteil des Durchmessermaßes der zentralen Erhöhung 18 erstreckt. Die Nadel 31 liegt in der durch die Querkanäle 22 des Trägers 17 aufgespannten Ebene ein. In einseitiger Verlängerung zur Ausnehmung 32 geht diese über in eine gleichgerichtete Radialbohrung 33, welche durchmessermäßig angepasst ist an den Außendurchmesser der Nadel 31. In dieser Radialbohrung 33 liegt die Nadel 31 geführt ein, wobei weiter die Radialbohrung 33 in Umfangsrichtung betrachtet mittig eine der Radialerweiterungen 19 der Erhöhung 18 durchsetzt, zur verlängerten Ausrichtung zu einem Querkanal 22 des Trägers 17 (vgl. bspw. Fig. 10). Auf einen Grundriss bezogen weist die Nadel 31 mit ihrer Spitze 34 die Radialbohrung 33 durchsetzend radial nach außen in Richtung auf das Mundstück 6, dies weiter mit Betrachtung eines Grundrisses in Überdeckung zu einer Mundstück-Kanalachse y, die weiter senkrecht ausgerichtet ist zur Spenderachse x.

Die Nadel 31 weist einen Durchmesser auf, der in dem dargestellten Ausführungsbeispiel etwa dem halben Radius einer Kuppel 29 entspricht. Weiter ist die ebenmäßige Anordnung der Nadel 31 so gewählt, dass die kanülenartig ausgespitzte, wenngleich massiv ausgebildete Nadelspitze 34 die Kuppel 29 in Durchmesserrichtung, parallel ausgerichtet zur Aluminiumfolienschicht 28 durchsetzt zur Bildung zweier gegenüberliegender, entsprechend koaxial zur Nadelachse und darüber hinaus auch zur Querkanalachse ausgerichteter Löcher. Die Aluminiumfolienschicht 28 bleibt hiervon unbeeinflusst.

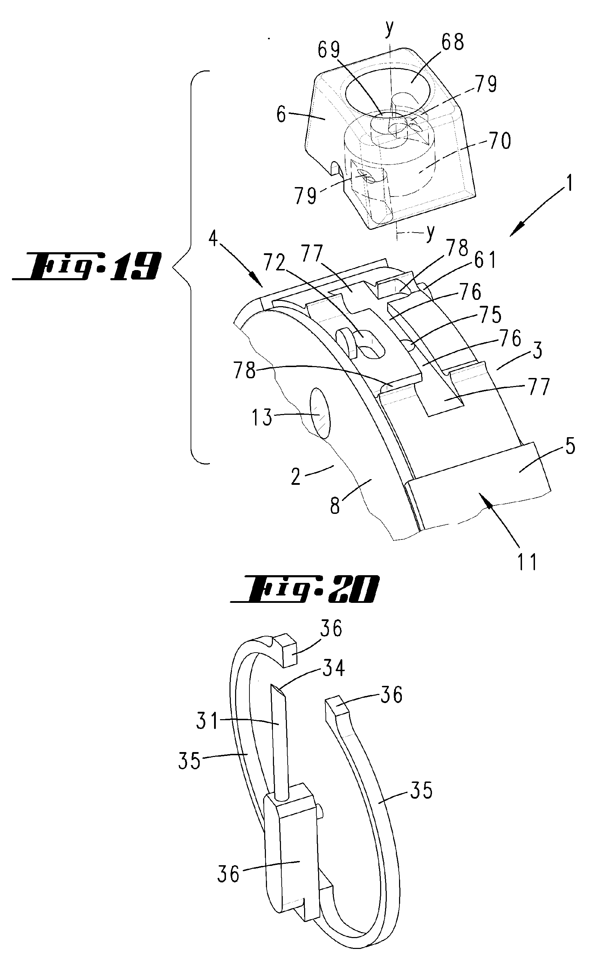

Die Nadel 31 ist fußseitig, d. h. im Bereich des der Spitze 34 abgewandten Endes mit angeformten, insbesondere im Kunststoffspritzverfahren einteilig mit der Nadel 31 gebildeten Federarmen 35 versehen. Wie in Fig. 20 zu erkennen, wurzeln diese Federarme 35 im Bereich eines auch die Nadel 31 tragenden Grundkörpers 36. In einem Grundriss betrachtet gehen von diesem Grundkörper 36 beidseitig gleichmäßig die Federarme 35 aus, die jeweils halbkreisringförmig nach außen gewölbt sind, zur Bildung von freien Federenden 37, die in unbelastetem Zustand gemäß der Einzeldarstellung in Fig. 20 mit Bezug auf einen Grundriss beidseitig benachbart zu der Nadelspitze 34 platziert sind. Mittels dieser Federenden 37 erfolgt eine Abstützung an wandungsinnenseitig an der Mantelwand 5, zugeordnet dem Bereich des Mundstückes 6 vorgesehenen Abstützschultern 38, die weiter von einteilig hieraus geformten Deckenabschnitten 39 überfangen sind, zur Sicherung der Federenden 37.

Zufolge dieser Ausgestaltung erfolgt bei einer Nadelvorverlagerung in Richtung auf das Mundstück 6, zum Durchstoßen der zugeordneten Kavität 26 mit der Nadelspitze 34 eine Spannung der Federarme 35, zufolge welcher Federspannung ein selbsttätiges Rückverlagern der Nadel 31 in die Ausgangsstellung erreicht werden kann. Diese rückwärtige Grundstellung ist anschlagbegrenzt, durch Anschlagen des Feder-Grundkörpers 36 an dem im Mundstück 6 abgewandten Ende der Ausnehmung 32.

Die willensbetonte Vorverlagerung der Nadel 31 zum Durchstoßen der zugeordneten Kuppel 29 erfolgt über eine Hebelanordnung. Diese besteht im Wesentlichen aus einem hebelartigen Schleppteil 40 und einem scheibenartigen Betätigungselement 41.

Das plan auf dem Nadel-Grundkörper 36 aufliegende Schleppteil 40 ist zugeordnet einem Randbereich des Mittelteils 11 an diesem angelenkt, wozu aus dem Mittelteil 11 ein Schwenkzapfen 42 auswächst. Um diesen ist das Schleppteil 40 schwenkverlagerbar. Anderenends weist das Schleppteil 40 eine ausgeformte Schleppnase 43 auf zur Zusammenwirkung mit dem Betätigungselement 41. Im Kreuzungsbereich zu der quer zum Schleppteil 40 ausgerichteten Nadel 31 ist das Schleppteil 40 mit einer Langlochausnehmung 44 versehen, in welche ein an dem Grundkörper 36 ausgebildeter, parallel zum Schwenkzapfen 42 ausgerichteter Mitnahmezapfen 45 eingreift.

Das scheibenförmige Betätigungselement 41 weist zugeordnet einem Randabschnitt 2 jeweils in Umfangsrichtung wirkende Betätigungsabschnitte auf, welche in Wechselwirkung stehen. Dies bedeutet, dass bei Nutzung des einen Betätigungsabschnitts der andere Betätigungsabschnitt inaktiv ist und umgekehrt. Diese Betätigungsabschnitte 46, 47 sind jeweils durch einen radialen Einschnitt und einen sich daran anschließenden kreisbogenförmigen Einschnitt freigestellt. Entsprechend sind die bevorzugt im Kunststoffspritzverfahren herausgeformten Betätigungsabschnitt 46 und 47 des Betätigungselements 41 senkrecht zur Flächenerstreckung des Betätigungselements 41 ausweichbar gestaltet, dies weiter unter materialbedingter Federbelastung der Abschnitte in die Grundstellung, d. h. in Richtung auf die gemeinsame Ebene von Abschnitten und Betätigungselement 41.

Die beiden Betätigungsabschnitte 46 und 47 gehen von einem gemeinsamen Wurzelbereich 48 aus; erstrecken sich von diesem ausgehend in entgegengesetzte Umlaufrichtungen. An diesem Wurzelabschnitt 48 ist zudem eine Handhabe 49 angeformt. Diese durchgreift eine entsprechend positionierte Langlochausnehmung 50 der Mantelwand 5 des Gehäuse-Mittelteils 11, zufolge dessen die Handhabe 49 nach außen ragt und somit frei zugänglich ist. Die Handhabe 49 liegt hierbei in einem durchmesserverringerten Mittenbereich der Mantelwand 5 ein, so dass die Handhabe 49 nicht bzw. nicht wesentlich über die Gesamtkontur des Spenders 1 bzw. des Spendergehäuses 4 überragt und dennoch leicht zu erfassen ist.

Der mit Bezug auf eine Draufsicht auf das in einer Ebene über dem Schleppteil 40 angeordnete Betätigungselement 41 entgegen der Uhrzeigerrichtung frei auskragende Betätigungsabschnitt 46 steht in einer Grundstellung in Formschlusseingriff zu dem Schleppteil 40. Hierzu weist der Betätigungsabschnitt 46 unterseitig, dem Schleppteil 40 zugewandt eine Mitnehmernase 51 auf. Die gegen der Uhrzeigerrichtung gerichtete Stirnfläche erstreckt sich in Mitnahmestellung im Wesentlichen senkrecht zur Erstreckungsebene des Betätigungselements 41 und tritt gegen die gleichgerichtete, zugewandte Fläche der schleppteilseitigen Schleppnase 43.

Der von dem Wurzelabschnitt 48 sich in Uhrzeigerrichtung frei erstreckende Betätigungsabschnitt 47 formt endseitig eine nach unten in Richtung auf den Blisterträger 17 weisenden Mitnahmefinger 53 auf. Dieser durchsetzt die Ebene des Gehäuse-Mittelteils 11 im Bereich einer aus dem Zwischenboden10 herausgeformten randnahen Ausnehmung 52.

Der Mitnahmefinger 53 ist stirnseitig, d. h. in Uhrzeigerrichtung betrachtet vorne mit einer senkrecht zur Betätigungselementebene verlaufenden Fläche versehen, während die hierzu rückwärtige, in Uhrzeigerrichtung wirkende Fläche eine Auflaufschräge ausformt.

Nach radial innen weisend ist an dem Mitnahmefinger 53 bzw. an dem diesen tragenden Betätigungsabschnitt 47 ein Ausrückzapfen 54 angeformt. Dieser ragt frei in den zur Bildung des Betätigungsabschnitts 47 belassenen Freischnitt.

Der Mitnahmefinger 53 des Betätigungselements 41 bildet zusammen mit einem ortsfest an dem Gehäuse-Mittelteil 11 im radial äußeren Bereich des Zwischenbodens 10 nahe der Innenwandung der Mantelwand 5 festgelegten Rückhaltefinger 55 ein Schrittschaltwerk 56 zur schrittweisen Verlagerung des Trägers 17 und des hierin aufgenommenen Blisters 25.

Das scheibenartige Betätigungselement 41 ist über die Handhabe 49 betätigbar zur Drehverlagerung geeignet.

Koaxial ausgerichtet zu einer zentralen Bohrung 57 ist aus dem Flächenteil des Betätigungselements 41 eine kreisabschnittförmige Langlochausnehmung 59 gebildet, in welche zur beidseitigen Anschlagbegrenzung für die Verschwenkbarkeit des Betätigungselements 41 ein gehäuseinnenseitig an der vorderen Deckwandung 8 angeformter Zapfen 58 greift. Dieser erstreckt sich längenmäßig bis zum Zwischenboden und formt diesem zugewandt in Überdeckung zu einer Durchgangsöffnung in dem Zwischenboden eine Festlegungshülse aus. In diese greift formschlüssig ein den Zwischenboden 10 durchsetzender Stift 60 ein, zur betrieblich nicht lösbaren Festlegung der Deckwandung 8.

Diametral gegenüberliegend zur Handhabe 49 trägt das Betätigungselement 41 einen zeigerartigen Sperrnocken 61. Dieser durchgreift einen zwischen der Mantelwand 5 und der vorderen Deckwandung 8 randseitig belassenen Schlitz und überfängt mit einem nach radial innen weisenden Übergreifabschnitt einen in diesem Bereich, umfangsmäßig in der Länge angepasst an den möglichen Verschenkweg des Betätigungselements 41 dickenmäßig verjüngten Abschnitt der Deckwandung 8.

Der zeigerartige Sperrnocken 61 gibt dem Benutzer einen visuellen Hinweis auf die derzeitige Betriebsstellung des Spenders 1. Weiter wirkt der Sperrnocken 61 im Zusammenspiel mit der Verschlusskappe 7 des Mundstücks 6 als Sicherung gegen ein Verschließen des Mundstückes 6 in einer Nicht-Bereitschaftsstellung.

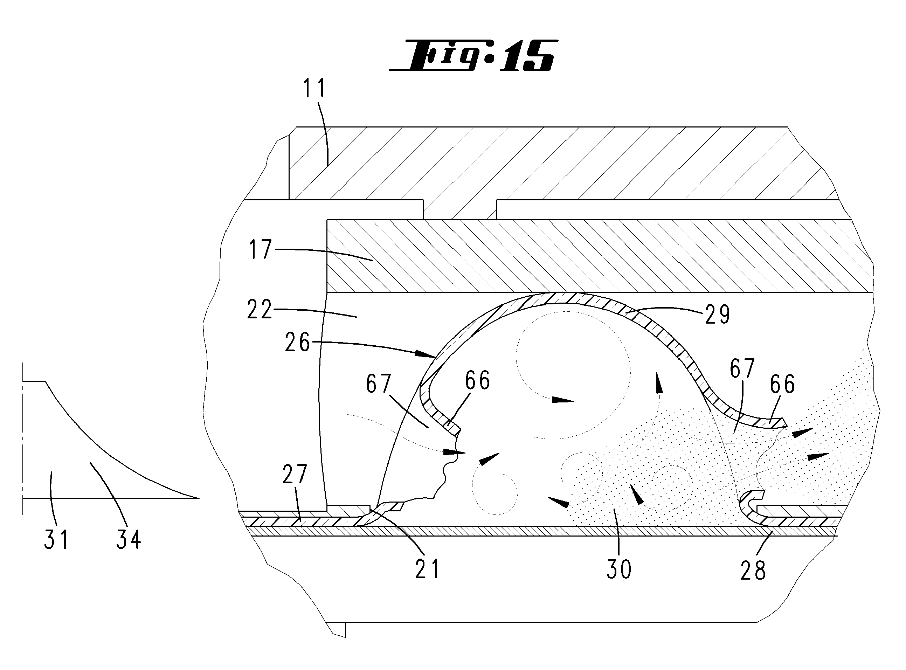

Aus einer Bereitschaftsstellung heraus, in welcher eine unversehrte, gefüllte Kavität der von der Nadelspitze 34 durchsetzten Radialbohrung 33 des Zwischenbodens 10 zugeordnet ist, erfolgt eine Vorbereitung zur Inhalation durch eine Lochung der entsprechenden Kuppel 29. Hierzu erfasst der Benutzer die Handhabe 49 und verlagert diese mit Bezug auf eine Draufsicht auf die vordere Deckwandung 8 des Spenders 1 entgegen Uhrzeigerrichtung. Hiermit einhergehend wird das Betätigungselement 41 um die Achse x drehverlagert, unter Mitschleppen des Schleppteils 40 aufgrund der im Zuge der Drehverlagerung bestehenden Schleppverbindung zwischen Mitnehmernase 51 des Betätigungsabschnitts 46 und Schleppnase 43 des Schleppteils 40. Bedingt durch die der Schleppverbindung gegenüberliegenden Drehlagerung des Schleppteils 40 wandert der Mitnahmezapfen 45 mitverlagert über die Langlochausnehmung 44 quer zur Ausrichtung der Achse x, was zu einer Vorverlagerung der angeformten Nadel 31 führt. Diese dringt hierbei in den zugeordneten Querkanal 22 des Trägers 17. Unter weiterer Vorverlagerung durchstößt die Nadelspitze 34 zunächst einen ersten Kuppel-Wandungsabschnitt und nach Durchtauchen durch die Kuppel 29 den hierzu diametral gegenüberliegenden Wandungsabschnitt. Die Durchstoßung der Kuppelwandung erfolgt hierbei nahe der Kuppel-Durchmesserlinie, d. h. nahe ihrer Anbindung an die Aluminiumfolienschicht 28 bzw. nahe ihrer randseitig umlaufenden Abstützung an dem Bohrungsrand des Trägers 17 (vgl. hierzu Fig. 15).

Der bis zur vollständigen Durchstoßung der Kuppel 29 die Schleppnase 43 des Schleppteils 40 mitnehmende Betätigungsabschnitt 46 erreicht hiernach mit seiner freien, eine Auflaufschräge ausformenden Stirnfläche 62 einen innenseitig von der Mantelwand 5 radial nach innen einragenden Ausrückzapfen 63. Dieser führt im Zuge einer weiteren Drehverlagerung des Betätigungselements 41 entgegen Uhrzeigerrichtung in Zusammenwirkung mit der Steuerfläche 62 zu einem vertikalen Ausrücken des Betätigungsabschnitts 46, nach welcher vollständigen Vertikalverlagerung die Mitnehmernase 51 die schleppteilseitige Schleppnase 43 freigibt.

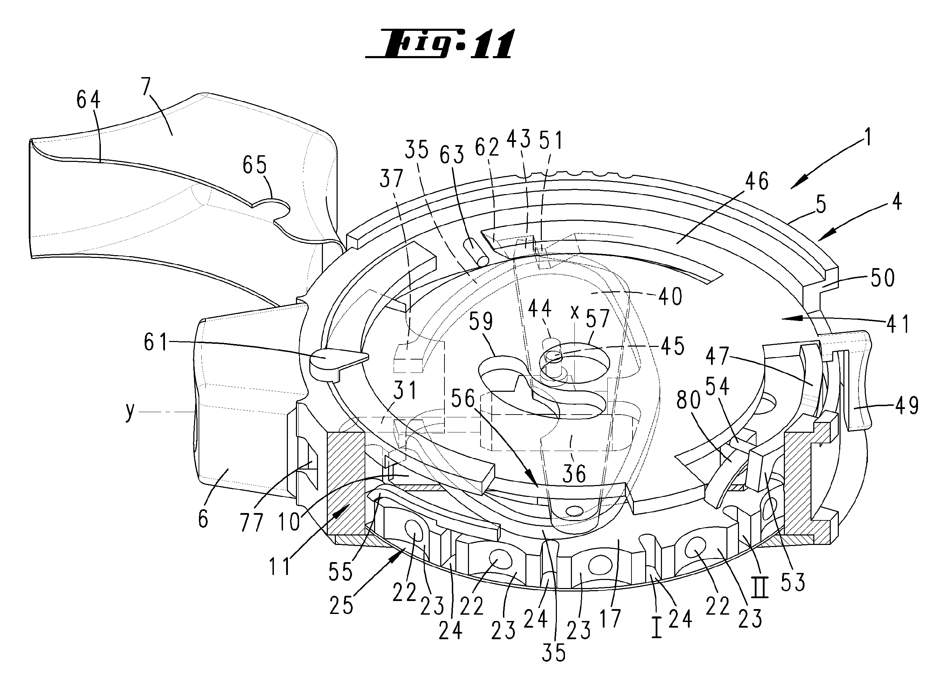

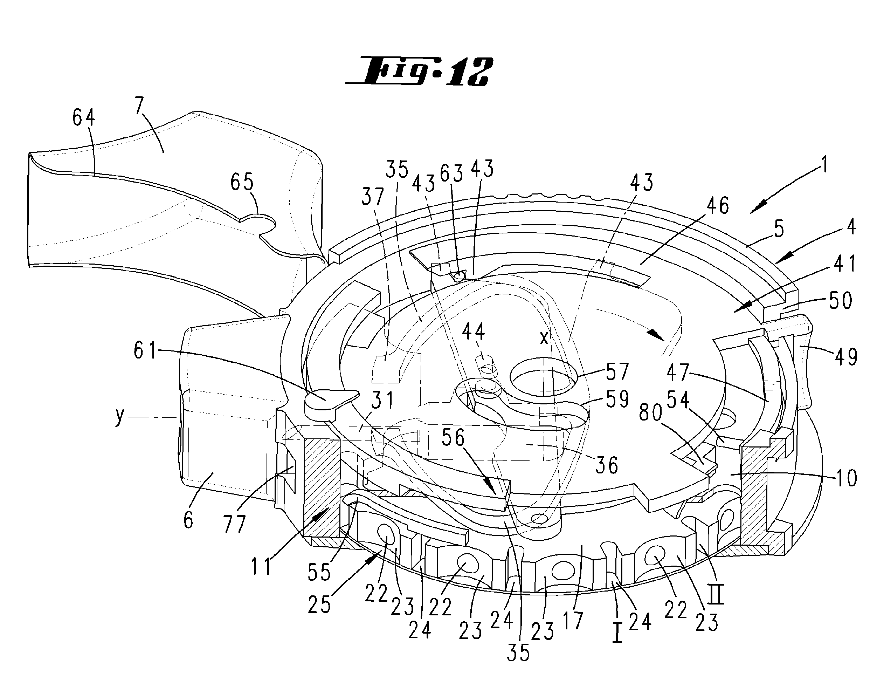

Die im Zuge der Nadelvorverlagerung gespannten Federarme 35 (vgl. Fig. 11 und 12) führen nach Aufhebung der Schleppverbindung zwischen Betätigungsabschnitt 46 und Schleppteil 40 zu einer ausgelösten, selbsttätigen Rückverlagerung der Nadel 31 in ihre Ausgangsstellung, dies unter Entspannung der Federarme 35.

Die Durchstoßung der Kuppel 29 erfolgt noch vor Erreichen der anschlagbegrenzten Drehendstellung des Betätigungselements 41. Entsprechend schnellt die Nadel 31 federunterstützt mit Erreichen oder kurz vor Erreichen dieser Endstellung zurück. Diese anschlagbegrenzte Endstellung ist auch für den Benutzer ersichtlich. Der zeigerartige Sperrnocken 61 nimmt eine gegenüber der Grundstellung, entsprechend dem Verschwenkweg von Handhabe 49 und Betätigungselement 41 veränderte Stellung ein. In dieser Stellung ist ein Schließen der Verschlusskappe 7 nicht möglich, da diese in dieser Stellung mit dem in diesem Fall eine Sperrschulter 64 ausformenden Kappenrand gegen den Sperrnocken 61 trifft. Ein Schließen der Verschlusskappe 7 ist nur in der entgegengesetzten Endstellung möglich. In dieser Stellung wirkt der Sperrnocken 61 mit einer randoffenen Ausnehmung 65 des Kappenrandes zusammen.

Etwaige beim Durchstoßen der Kuppel 29 mit der Nadel 31 aus der Kavität 26 nach außen durchgedrückte Massepartikel gehen bei diesem Vorgang nicht für den folgenden Inhalationsvorgang verloren, da diese in den Querkanal 22 eingebracht werden, welcher im Zuge der Inhalation Teil des Saugluftstromkanals wird. Im Zuge der selbsttätigen, federunterstützten Rückverlagerung der Nadel 43 wird über die in Durchstoßrichtung sich einstellenden Lappen 66 im Bereich der Kuppellöcher 67 ein Abstreifen der Nadeloberfläche erreicht.

Die in der Kavität 26 bevorratete, portionierte Masse 30 liegt nunmehr frei im Saugluftstrom-Kanal, bereit zur Inhalation. Diese Inhalation erfolgt bei dem dargestellten Spender oral. Hierzu weist das Mundstück 6 zentral koaxial zur Achse y einen sich nach außen radial erweiternden Austrittstrichter 68 auf. Dieser geht zunächst über in einen zentralen durchmesserverringerten Zwischenabschnitt 69, der in einer wiederum radial erweiterten Wirbelkammer 70 mündet. Diese Wirbelkammer 70 weist einen Kammerboden 71 auf. Dieser ist zugeordnet einem in Verlängerung zu der Radialbohrung 33 des Zwischenbodens 10 in der Mantelwand 5 des Gehäuses 4 ausgebildeten Saugkanalabschnitt 72 mit einer fensterartigen Öffnung 73 versehen.

Öffnung 73, Saugkanalabschnitt 72 und der in Wirkstellung verbrachte Querkanal 22 des Trägers 17 sind hintereinandergeschaltet angeordnet, bilden entsprechend einen geradlinigen parallel zur Achse y verlaufenden Saugkanal. Dieser geht fußseitig, d. h. abgewandt dem Mundstück 6, weiter zugeordnet der von der Nadel 31 dichtend durchsetzten Radialbohrung 33, über in einen in Parallelausrichtung zur Achse x verlaufenden, in dem Zwischenboden 10 ausgebildeten Umlenkkanal 74, der wiederum übergeht in einen zurück in Richtung auf das Mundstück 6 gerichteten Kanalabschnitt 75. Dieser ist gleichfalls geradlinig ausgerichtet und erstreckt sich in einer mit Bezug auf die Achse x mittleren Ebene; ausgebildet aus dem insgesamt massiven Zwischenboden 10.

Der Kanalabschnitt 75 und der sich aus dem Saugkanalabschnitt 72 und im Wesentlichen dem Querkanal 22 zusammensetzende weitere Kanalabschnitt sind übereinanderliegend angeordnet und über eine 180°-Umlenkung miteinander verbunden.

Der Kanalabschnitt 75 mündet überdeckt von dem Kammerboden 71 in beidseitig jeweils etwa in Umlaufrichtung der Mantelwand 5 erstreckende Ansaugkanalabschnitte 76. Diese erstrecken sich entsprechend unterhalb des Mundstücks 6 quer zu dem Kanalabschnitt 75 und enden, wie beispielsweise aus der Darstellung in Fig. 4 zu erkennen, seitlich des Mundstückes 6 frei die Mantelwand 5 durchsetzend nach außen. Über diese Ansaugöffnungen 77 wird im Zuge der Inhalation durch Einatmen Luft angesogen.

Wie weiter insbesondere aus der Darstellung in Fig. 19 zu erkennen, gehen von den Ansaugkanalabschnitten 76 jeweils Stichkanäle 78 aus. Diese stehen strömungstechnisch mit düsenartigen, tangential die Mantelwandung der Wirbelkammer 70 durchtretenden Bypass-Öffnungen 79 in Verbindung.

Die Inhalation erfolgt nach Aufstoßen der Kuppel 29 durch Ansaugen über das Mundstück 6. Hierbei wird Luft über die Ansaugöffnungen 77 und die quergerichteten Ansaugkanalabschnitte 76 zunächst über den Kanalabschnitt 75 nach radial innen des Spenders 1 gesaugt, hiernach unter 180°-Umlenkung durch den zugeordneten Querkanal 22 des Trägers 17. Die den Querkanal 22 durchströmende Luft wird durch die geöffnete Kuppel 29 geführt, aus welcher diese unter günstiger Verwirbelung innerhalb der Kuppel 29 die Masseteilchen austrägt und über den Saugkanalabschnitt 22, Wirbelkammer 70 und Zwischenabschnitt 69 zum Austrittstrichter 68 transportiert (Pfeile a), wobei weiter im Bereich der Wirbelkammer 70 über die Bypass-Öffnungen 79 eine wandungsseitige Verwirbelung insbesondere großer Massepartikel erreicht wird. Die zusätzlichen Horizontalwirbel (Pfeile b) sorgen für eine gute Verteilung.

Durch die findungsgemäße Öffnung der Kuppel 29 durch Durchstoßen diametral gegenüberliegender Wandungsabschnitte der Kuppel ohne Beschädigung der den Boden bildenden Aluminiumfolieschicht 28 wird eine vollständige Entleerung erreicht und somit weiter eine portionsgerechte Inhalation gesichert.

Wie erwähnt, ist aus dieser Stellung heraus kein Schließen der Mundstück-Verschlusskappe 7 erreichbar. Dem Benutzer ist hierdurch ein Sicherungsmittel gegeben, das ihm einen Hinweis darauf gibt, dass zwar die Kuppel gelocht, jedoch entweder der Inhalationsvorgang noch nicht durchgeführt wurde oder der Spender 1 nicht für den nächsten Inhalationsvorgang vorbereitet ist.

Diese Vorbereitung erfolgt durch Dreh-Rückverlagerung des Betätigungselements 41 mittels der Handhabe 49 entgegen der für die Lochung notwendigen Drehrichtung, also mit Bezug auf die beschriebene Sichtweise nunmehr in Uhrzeigerrichtung. Hierbei kommt das Schrittschaltwerk 56 zum Einsatz. Deren Finger 55 und 53 wirken mit den randseitigen Ausklinkungen 24 des Trägers 17 zusammen, wobei der Rückhaltefinger 55 entsprechend seiner Auslegung nur eine Verdrehung des Trägers 17 in dem dargestellten Ausführungsbeispiel in Uhrzeigerrichtung zulässt.

Die schrittweise Verlagerung des Trägers 17 wird über den Mitnahmefinger 53 erreicht. Dieser wird im Zuge der Nadelvorverlagerung, d. h. bei Verdrehung des Betätigungselements 41 entgegen Uhrzeigerrichtung über seinen Ausrückzapfen 54 vertikal nach oben ausgelenkt. Hierzu ist an dem Zwischenboden 10 eine Auflaufschräge 80 ausgeformt. Über diese wird der Mitnahmefinger 53 in eine Ebene oberhalb des Trägers 17 verbracht, wobei im weiteren Zuge der Drehverlagerung entgegen Uhrzeigerrichtung der Ausrückzapfen 54 die Auflaufschräge 80 verlässt, was das vertikale Abfallen des Mitnahmefingers 53 auf einen den Träger 17 überdeckenden Zwischenbodenabschnitt bewirkt.

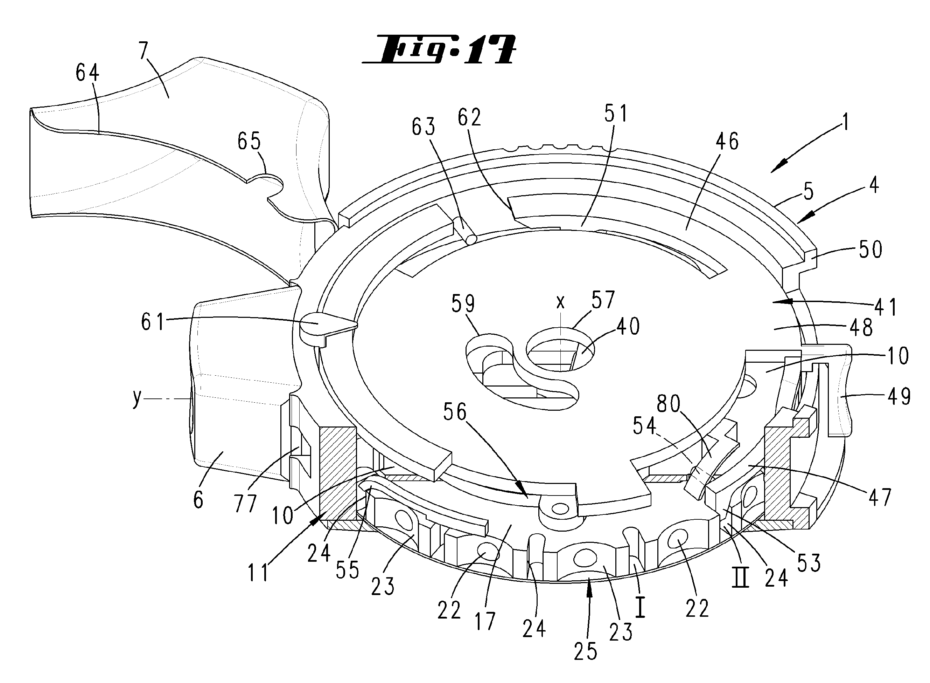

Erst die Drehrückverlagerung des Betätigungselements 41 in Uhrzeigerrichtung führt zu einem Verlassen der Abstützung des Mitnahmefingers 53 auf dem Zwischenbodenabschnitt. Der Mitnahmefinger 53 fällt bedingt durch die entsprechende Ausgestaltung des Betätigungsabschnitts 47 federunterstützt vertikal ab in Richtung auf den Träger 17, zum Einfallen in die in Uhrzeigerrichtung nächste Ausklinkung 24. Unterstützt wird dieser Fingereingriff noch durch Beaufschlagung des angeformten Ausrückzapfens 54 von oben durch die Unterseite des die Auflaufschräge 80 oberseitig ausbildenden Abschnitts (vgl. Fig. 17). Dieser die Auflaufschräge 80 formende Abschnitt ist federnd ausweichbar gebildet, so dass im weiteren Zuge der Verdrehung der den Träger 17 nunmehr mitschleppende Mitnahmefinger 53 mit seinem Ausrückzapfen 54 unter diesem Abschnitt durchtauchen kann, wonach der Ausrückzapfen 54 wiederum für die nächste Verlagerung entgegen Uhrzeigerrichtung der Auflaufschrägen 80 zugewandt ausgerichtet ist.

Nach dieser Schrittverlagerung des Trägers 17 (beispielhaft an den Schrittpositionen I und II dargestellt) ist die nächste Kavität dem Saugkanalabschnitt 72 sowie der Nadel 31 zugeordnet.

Dies ist auch die Stellung, in welcher ein Schließen der Mundstückkappe 7 erreicht werden kann.

Die Mitnehmernase 51 liegt nach Überlaufen der schleppteilseitigen Schleppnase 43 wieder in einer Mitnahmestellung zu der Schleppnase 43; vorbereitet für den nächsten Lochungsvorgang.

## Patentansprüche

1. Zur Verwendung in Medikamenten-Spendern geeignete Blisterpackung (25) mit auf einer Aluminiumschicht (28) kreisförmig auflaminierten, kuppelförmigen Kavität (26), die mittels einer Durchstoß-Nadel (31) öffenbar sind, **dadurch gekennzeichnet, dass** die die Kavitäten (26) tragende Alu-Schicht (28) die Gestalt eines Ringstreifens hat.

2. Spender mit Blisterpackung gemäß Anspruch 1, **dadurch gekennzeichnet, dass** die Nadel (31) die Kavitäten-Kuppel (29) vom Zentrum des Ringstreifens her auf der Durchmesserlinie durchstößt.
